(19) Europäisches Patentamt
European Patent Office
Office européen des brevets

(11) Veröffentlichungsnummer: **0 065 286**
**B1**

(12) # EUROPÄISCHE PATENTSCHRIFT

(45) Veröffentlichungstag der Patentschrift: **08.10.86**

(51) Int. Cl.⁴: **A 23 J 1/00**, C 07 K 3/12, C 12 N 9/00

(21) Anmeldenummer: **82104186.0**

(22) Anmeldetag: **12.05.82**

(54) **Verfahren zum Reinigen bzw. Anreichern von biologisch aktiven Proteinen und hierzu geeignetes Mittel.**

(30) Priorität: **15.05.81 DE 3119453**

(43) Veröffentlichungstag der Anmeldung: **24.11.82 Patentblatt 82/47**

(45) Bekanntmachung des Hinweises auf die Patenterteilung: **08.10.86 Patentblatt 86/41**

(84) Benannte Vertragsstaaten: **AT BE CH DE FR GB IT LI LU NL SE**

(56) Entgegenhaltungen:
**EP - A - 0 009 781**
**WO - A - 79/00541**

**CHEMICAL ABSTRACTS, Band 86, Nr. 17, 25. April 1977, Seite 147, Nr. 116354y, Columbus, Ohio, USA, I.I. IVANOV et al.: "Absence of the ability of highly purified myosin preparations to form contractile complexes with DNA"**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Vetter, Hellmuth, Dr., Kreuzeckstrasse 17, D-8132 Tutzing (DE)**
Erfinder: **Scheibe, Peter, Dr., Schremelstrasse 39, D-8000 München 60 (DE)**
Erfinder: **Thum, Waldemar, von Kühlmann-Strasse 11, D-8132 Tutzing (DE)**
Erfinder: **Näher, Gotthilf, Dr., Zugspitzstrasse 22, D-8132 Tutzing (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 065 286 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zum Reinigen bzw. Anreichern von biologisch aktiven Proteinen.

Für die Anreicherung von biologisch aktiven Proteinen sind bereits mehrere Anreicherungsmittel bzw. -verfahren bekannt. Beispiele für geeignete Mittel sind Salze, wie Ammoniumsulfat, organische Lösungsmittel, wie Aceton, organische Gele, Aktivkohle, Austauscherchromatographie, Molekularsiebe, Polyethylenimine und einige andere. In Anbetracht der ausserordentlichen Vielfalt von natürlich vorkommenden aktiven Proteinen und ihrer Begleitstoffe macht sich jedoch häufig das Fehlen von Variationsmöglichkeiten bei der Auftrennung bzw. Reinigung sehr nachteilig bemerkbar. Aus der an sich schon begrenzten Anzahl von Verfahren und Mitteln zur Reinigung und Anreicherung von Proteinen erweisen sich meist nur ganz wenige in einem speziellen Fall als brauchbar.

Dies hat zur Folge, dass vielfach die gleichen Reinigungsschritte wiederholt werden müssen, um eine einigermassen befriedigende Abtrennung von Begleitstoffen zu erzielen. Zumeist sind die erzielten Anreicherungsgrade zudem gering und die spätere Entfernung des zur Anreicherung verwendeten Mittels erfordert besondere Massnahmen, wie Eindampfen grosser Flüssigkeitsvolumina und dergleichen. Die verschiedenen bekannten Verfahren zur Reinigung bzw. Anreicherung von biologisch aktiven Proteinen weisen daher im grossen und ganzen stets die gleichen Verfahrensschritte auf, die sich in grosser Ähnlichkeit, wenn auch in unterschiedlicher Anordnung, wiederholen.

Aus WO-A 7 900 541 und EP-A 0 009 781 sind Verfahren zur Affinitätschromatographie von Proteinen unter Verwendung von Farbstoffen bekannt.

Es besteht daher Bedarf an zusätzlichen Verfahren zum Reinigen und Anreichern von biologisch aktiven Proteinen, welche die zur Verfügung stehenden Variationsmöglichkeiten verbessern und die Proteinanreicherung wirksamer gestalten. Aufgabe der Erfindung ist daher die Schaffung eines weiteren Verfahrens und Mittels zum Reinigen bzw. Anreichern von biologisch aktiven Proteinen, welches die biologische Aktivität der Proteine möglichst wenig beeinträchtigt und die bestehenden Variationsmöglichkeiten erweitert.

Gelöst wird diese Aufgabe erfindungsgemäss durch ein Verfahren zum Reinigen bzw. Anreichern von biologisch aktiven Proteinen durch Zusatz eines selektiven Fällungsmittels, welches dadurch gekennzeichnet ist, dass man eine wässrige Lösung, die das gewünschte Protein enthält, bei einem pH-Wert unter dessen isoelektrischem Punkt mit einer solchen Menge eines arylsulfonsauren Azofarbstoffs mit zwei Sulfonatgruppen, die durch 8 bis 10 in Resonanzbeziehung stehende Doppelbindungen, unter welchen sich wenigstens eine N = N-Doppelbindung befindet, voneinander entfernt sind, versetzt, bis zumindest der grösste Teil der biologischen Aktivität des gewünschten Enzyms sich im Niederschlag befindet, den Niederschlag abtrennt und das aktive Protein bei einem pH-Wert über seinem isoelektrischen Punkt wieder in Lösung bringt.

Es ist zwar bekannt, dass viele Farbstoffe mit Proteinen eine Wechselwirkung eingehen. Soweit jedoch Fällungen hierbei auftreten, geht normalerweise die biologische Aktivität des im Fällungskomplex enthaltenen Proteins völlig verloren. Überraschenderweise tritt diese Denaturierung bei den oben definierten Azofarbstoffen nicht auf und ausserdem erfolgt die Fällung der verschiedenen Proteine sehr selektiv, so dass ein ausgezeichneter Reinigungs- und Anreicherungseffekt erzielt wird. Besonders überraschend ist, dass das erfindungsgemässe Verfahren nur wenig von Fremdsalzen abhängig ist und sich daher zur Anwendung auf rohe Zellextrakte eignet. Es ist daher in vielen Fällen möglich, selektiv aus rohen Zellextrakten das gesuchte biologisch aktive Protein zu fällen und vom Grossteil der verunreinigenden Fremdproteine und sonstigen Zellextrakt-Inhaltsstoffen abzutrennen. Desgleichen ist die direkte Fällung aus Fermentationsbrühen möglich. Da die Sulfonsäuregruppen des Fällungsmittels bis herab zu etwa pH 2 voll ionisiert sind, hängt der für die Fällung optimale pH-Wert praktisch ausschliesslich von der Ionisierbarkeit und pH-Stabilität des jeweiligen aktiven Proteins ab. Im allgemeinen eignet sich das Verfahren bei solchen Proteinen, deren biologische Aktivität auch unterhalb ihres isoelektrischen Punktes noch aufrechterhalten bleibt. Diese Voraussetzung ist im allgemeinen gegeben, wenn sich das aktive Protein an Kationenaustauscher ohne wesentlichen Aktivitätsverlust adsorbieren lässt.

Dies kann in einem einfachen Vorversuch rasch festgestellt werden.

Unter biologisch aktiven Proteinen im Sinne der Erfindung werden vorzugsweise die enzymatisch aktiven und die immunologisch aktiven Proteine verstanden.

Das Verfahren eignet sich jedoch auch für andere biologisch aktive Proteine, beispielsweise Hormone, Toxine, Immunglobuline und dergleichen.

Beispiele für enzymatisch aktive Proteine, die sich für das erfindungsgemässe Verfahren eignen, sind ADH aus Leber, Aldolase, α-Amylase aus Pilzen bzw. Pankreas, Ascorbatoxidase, Bromelain, α-Bungarotoxin, β-Bungarotoxin, Cholesterinesterase, Cholesterinoxidase, Carboxypeptidase-B, Chymotrypsin, Diaphorase, Fumarase, α-Galactosidase aus Kaffeebohnen oder aus Hefe, Glucose-6-phosphat-dehydrogenase aus Hefe, Glutathionreduktase, Glycerin-3-phosphat-dehydrogenase, Oxalatoxidase, Papain, Phospholipase A2, Peroxidase, Penicillinamidase, 6-Phosphoglukonat-dehydrogenase, Trypsin, Triosephosphat-isomerase und Uridin-diphosphoglucosepyrophosphorylase. Zahlreiche andere Enzyme eignen sich in gleicher Weise.

Vorzugsweise verwendet man erfindungsgemäss als Azofarbstoff 3,3′-(4,4′-Biphenylylenbis(azo))bis(4-amino-1-naphthalinsulfonsäure)

oder ein durch ein oder mehrere Niedrigalkyl- oder Niedrigalkoxygruppen substituiertes Derivat davon.

Die Verbindungen dieser bevorzugten Gruppe weisen folgende allgemeine Formel auf:

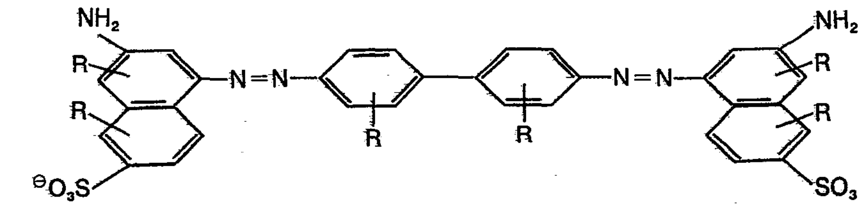

worin jedes R unabhängig voneinander ein Wasserstoffatom, $C_{1-4}$-Alkyl oder $C_{1-4}$-Alkoxy bedeutet.

Typische Beispiele für diese bevorzugte Gruppe sind die handelsüblichen Farbstoffe Kongorot (C.I. 22120) und Benzopurpurin 10B (C.I. 23560).

Eine weitere bevorzugte Gruppe von Azofarbstoffen besteht aus 2,2'[(Diazoamino)-di-p-phenylen]bis(7-benzothiazolsulfonsäure) und ihren ein oder mehrere Niedrigalkyl- oder/und Niedrigalkoxygruppen tragenden Derivate.

Die Verbindungen dieser bevorzugten Gruppe weisen folgende allgemeine Formel auf:

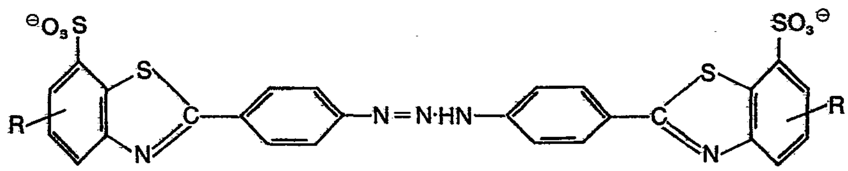

worin jedes R unabhängig voneinander ein Wasserstoffatom, $C_{1-4}$-Alkyl oder $C_{1-4}$-Alkoxy bedeutet.

Ein typisches Beispiel für diese bevorzugte Gruppe ist Thiazolgelb G (C.I. 19540).

Der Farbstoff wird in gelöster Form zugesetzt. Als gut geeignet erwiesen sich 0,05 bis 5%-ige Lösungen (in Abhängigkeit von der Löslichkeit des jeweiligen Azofarbstoffs), jedoch können auch stärker verdünnte Lösungen angewendet werden, obwohl hierdurch in der Regel unnötig viel Wasser zugeführt wird. Die geeignete Farbstoffmenge hängt von der Menge des zu fällenden Proteins ab. Im allgemeinen liegt sie zwischen etwa 0,01 und etwa 2,0% Gew./Vol., vorzugsweise zwischen 0,05 und 0,2% Farbstoff/Volumen Lösung.

Die pH-Bedingungen hängen, wie bereits erwähnt, vom isoelektrischen Punkt des aktiven Proteins ab. Im allgemeinen kommen pH-Werte zwischen etwa 2 und etwa 9 in Betracht.

Die Auflösung des Niederschlags erfordert, wie oben erwähnt, ein Anheben des pH-Werts über den isoelektrischen Punkt. Im allgemeinen wird man daher den abgetrennten Niederschlag mit einer Pufferlösung eines pH-Werts zwischen etwa 4 und etwa 10 wieder auflösen. Aus der so erhaltenen Lösung kann dann der Farbstoff nach üblichen Methoden entfernt werden. Beispiele hierfür sind: Fällung des Farbstoffs mittels Polykationen, Fällung des aktiven Proteins durch Zusatz von Salzen, wie Ammoniumsulfat, oder von organischen Lösungsmitteln wie Aceton, Adsorption des Farbstoffes an Anionenaustauscher, wie Diäthylaminoäthylgruppen enthaltende polymere Kohlehydrate oder hydrophobe Polymere, wie Polyvinyl-Polypyrrolidon, Dialyse oder Ultrafiltration. Geeignete Polykationen sind beispielsweise die Polyäthylenimine.

Der ausfallende aktive Protein-Azofarbstoff-Komplex erwies sich in allen untersuchten Fällen als gut filtrierbar. Da in vielen Fällen aufgrund der hohen Selektivität des Verfahrens der Fällung des gesuchten aktiven Proteins Fällungen anderer Proteine und Begleitsubstanzen vorangehen, erweist sich die leichte Entfernbarkeit als besonderer Vorteil für die Anreicherung. Die vor dem gesuchten Protein ausfallenden Substanzen lassen sich aufgrund dieser Eigenschaften leicht und vollständig abtrennen. Gleiches gilt auch für die Fällung des gesuchten aktiven Proteins selbst, welches wiederum leicht durch Filtrieren oder Zentrifugieren von den noch in Lösung befindlichen Verunreinigungen abgetrennt werden kann.

Die Erfindung weist zahlreiche Vorteile auf. Bereits erwähnt wurde die relativ geringe Beeinflussung der Fällung durch den Salzgehalt der Lösungen, so dass auch sehr unreine Lösungen, wie Fermentationsbrühen und Zellaufschlusslösungen, direkt nach dem erfindungsgemässen Verfahren behandelt werden können. Weiter kommt das erfindungsgemässe Verfahren mit geringen Mengen Fällungsmittel aus und erspart hierbei in vielen Fällen die bisher zur Fällung benötigten grossen Mengen organischer Lösungsmittel. In einem typischen Fall wurden bisher für die Vorreinigung eines Rohextraktes 60%-ige Aceton- bzw. Methanolkonzentrationen benötigt, was bei 1000 l Rohextrakt 1500 l organisches Lösungsmittel erfordert. Demgegenüber gelingt es erfindungsgemäss, die Fällung mit 1 kg Farbstoff zu erzielen und gleichzeitig noch eine weitaus bessere Reinigung, d.h. Abtrennung unerwünschter Begleitsubstanzen, zu erzielen.

Die folgenden Beispiele erläutern die Erfindung weiter.

Beispiel 1

Eine handelsübliche, lyophilisierte Rohlipase wurde in 0,01 M Natriumacetat, pH 3,6, auf eine Aktivität von etwa 650 U/ml gelöst. Dieser Lösung werden 0,3 mg Kongorot pro ml in Form einer 2%-igen wässrigen Lösung zugesetzt. Man erhält einen Niederschlag, der etwa 70% der Ausgangsaktivität enthält. Im Überstand ist nur noch eine geringe Restaktivität feststellbar. Der Niederschlag wird abfiltriert, mit dem gleichen Puffer nachgewaschen und mit Puffer, pH 5,0, wieder gelöst. Durch Zutropfen einer 10%-igen Polyäthyleniminlösung wird der Farbstoff quantitativ gefällt. Die gesamte Aktivität des Niederschlags befindet sich danach im Überstand.

Eine Wiederholung dieses Versuchs mit Benzopurpurin 10B ergibt praktisch gleiche Resultate.

Beispiel 2

Ein ascorbatoxidasehaltiger Rohextrakt in 0,01 M Kaliumphosphat/Acetat-Puffer, pH 4,6, mit einem Gehalt von etwa 20 U/ml Ascorbatoxidase wird mit 0,4 mg Kongorot/ml in Form einer 2%-igen wässrigen Lösung versetzt. Der entstehende Niederschlag wird abgetrennt. Im Überstand werden 1% Restaktivität gemessen. Der Niederschlag wird mit 0,05 M Kaliumphosphat-Puffer, pH 7,6, gelöst. Man erhält so etwa 60% der Ausgangsaktivität in der Lösung. Der Farbstoff lässt sich, wie in Beispiel 1 beschrieben, abtrennen.

Beispiel 3

Gereinigte Cholesterinesterase wird in 0,05 M Kaliumphosphat-Puffer, pH 4, auf eine Konzentration von etwa 75 U/ml aufgenommen. Der so erhaltenen Lösung wird 1 mg Kongorot pro ml Lösung zugesetzt und der gebildete Niederschlag abgetrennt. Im Überstand werden 1% der Ausgangsaktivität festgestellt, der Niederschlag enthält 99% der Aktivität. Nach Auflösung des Niederschlags in 0,1 M Kaliumphosphat-Puffer, pH 6, erhält man 100% der Aktivität des Niederschlags wieder in der Lösung.

Das Verfahren wurde wiederholt, wobei der Ausgangslösung 0,3 M Natriumchlorid zugesetzt waren. Die Ergebnisse blieben unverändert.

Beispiel 4

Vorgereinigte Peroxidase mit einer spezifischen Aktivität von ca. 100 U/mg Lyophilisat werden in 0,02 M Natriumacetat-Puffer, pH 3,5, gelöst zu einer Konzentration von 100 U/ml. Dieser Lösung werden 1 mg Benzopurpurin/ml in Form einer 1%-igen wässrigen Lösung zugesetzt. Der gebildete Niederschlag wird abgetrennt. Im Überstand verbleiben 5% der Ausgangsaktivität an POD. Der Niederschlag wird mit 0,1 M Kaliumphosphat-Puffer, pH 7,6, wieder aufgelöst. In der Lösung finden sich 86% der Ausgangsaktivität wieder.

Beispiel 5

Phospholipase $A_2$ in Form eines Rohextraktes aus Pankreas mit einem Salzgehalt von etwa 0,1 M wird mit Essigsäure auf pH 4,3 eingestellt. Diese Lösung enthält 110 U/ml mit einer spezifischen Aktivität von ca. 15 U/mg Protein. Die Lösung wird mit 2,2 mg Kongorot/ml in Form einer 2%-igen Lösung versetzt und der aufgetretene Niederschlag abzentrifugiert. Der Überstand enthielt 7% der Aktivität. Der Niederschlag wurde mit 0,1 M Kaliumphosphatpuffer pH 7,0 gelöst und durch Ausfällung des Farbstoffes mittels Polyäthylenimin geklärt. Der Überstand nach Zentrifugation enthielt 92% der ursprünglichen Aktivität mit einer spezifischen Aktivität von 55 U/mg Protein.

**Patentansprüche**

1. Verfahren zum Reinigen bzw. Anreichern von biologisch aktiven Proteinen durch Zusatz eines selektiven Fällungsmittels, dadurch gekennzeichnet, dass man eine wässrige Lösung, die das gewünschte Protein enthält, bei einem pH-Wert unter dessen isoelektrischem Punkt mit einer solchen Menge eines arylsulfonsauren Azofarbstoffs mit zwei Sulfonatgruppen, die durch 8 bis 10 in Resonanzbeziehung stehende Doppelbindungen, unter welchen sich wenigstens eine N = N-Doppelbindung befindet, voneinander entfernt sind, versetzt, bis zumindest der grösste Teil der biologischen Aktivität des gewünschten aktiven Proteins sich im Niederschlag befindet, den Niederschlag abtrennt und das aktive Protein bei einem pH-Wert über seinem isoelektrischen Punkt wieder in Lösung bringt.

2. Verfahren nach Anspruch 1, dadurch gekennzeichnet, dass man als Azofarbstoff 3,3′-(4,4′-Biphenylylen-bis(azo))-bis(4-amino-1-naphthalinsulfonsäure) oder ein durch ein oder mehrere Niedrigalkyl- oder Niedrigalkoxygruppen substituiertes Derivat desselben verwendet.

3. Verfahren nach Anspruch 2, dadurch gekennzeichnet, dass man Kongorot (C.I. 22120) oder Benzopurpurin-10B (C.I. 23560) verwendet.

4. Verfahren nach Anspruch 2, dadurch gekennzeichnet, dass man 2,2′-[(Diazoamino)-di-p-phenylen]-bis(7-benzothiazolsulfonsäure) oder ein eine oder mehrere Niedrigalkyl- oder/und Niedrigalkoxygruppen tragendes Derivat davon verwendet.

5. Verfahren nach Anspruch 4, dadurch gekennzeichnet, dass man Thiazolgelb G (C.I. 19540) verwendet.

6. Verfahren nach einem der vorhergehenden Ansprüche, dadurch gekennzeichnet, dass man 0,01 bis 2,0% Gew./Vol. Farbstoff zusetzt.

7. Verfahren nach einem der vorhergehenden Ansprüche, dadurch gekennzeichnet, dass man den Azofarbstoff in Form einer 0,05 bis 5%-igen Lösung zusetzt.

8. Verfahren nach einem der vorhergehenden Ansprüche, dadurch gekennzeichnet, dass man die Fällung bei pH-Werten zwischen 2 und 9 durchführt.

9. Verfahren nach einem der vorhergehenden Ansprüche, dadurch gekennzeichnet, dass man den abgetrennten Niederschlag mit Pufferlösung, pH 4,5 bis 10, auflöst.

10. Verfahren nach einem der vorhergehenden Ansprüche, dadurch gekennzeichnet, dass aus der aus dem Niederschlag erhaltenen Lösung der Farbstoff durch Zusatz von Polykationen oder Adsorbantien, durch Ausfällen des aktiven Proteins mittels organischen Lösemitteln oder Salzen oder durch Dialyse bzw. Ultrafiltration, vom aktiven Protein getrennt wird.

**Claims**

1. Process for the purification or enrichment of biologically active proteins by the addition of a selective precipitation agent, characterised in that one mixes an aqueous solution, which contains the desired protein, at a pH value below its isoelectric point with such an amount of an arylsulphonic acid azo dyestuff with two sulphonate groups, which are separated from one another by 8 to 10 double bonds standing in resonance relationship, amongst which there is present at least one N = N double bond, until at least the greater part of the biological activity of the desired active protein is present in the precipitate, separates off the precipitate and again brings the active protein into solution at a pH value above its isoeletric point.

2. Process according to claim 1, characterised in that, as azo dyestuff, one uses 3,3′-(4,4′-biphenylylene-bis-(azo))-bis-(4-amino-1-naphthalenesulphonic acid) or a derivative thereof substituted by one or more lower alkyl or lower alkoxy groups.

3. Process according to claim 2, characterised in that one uses Congo Red (C.I. 22120) or benzopurpurin 10B (C.I. 23560).

4. Process according to claim 2, characterised in that one uses 2,2′-[(diazoamino)-di-p-phenylene]-bis-(7-benzothiazolesulphonic acid) or a derivative thereof carrying one or more lower alkyl and/or lower alkoxy groups.

5. Process according to claim 4, characterised in that one uses thiazole yellow G (C.I. 19540).

6. Process according to one of the preceding claims, characterised in that one adds 0.01 to 2.0% wt./vol. of dyestuff.

7. Process according to one of the preceding claims, characterised in that one adds the azo dyestuff in the form of a 0.05 to 5% solution.

8. Process according to one of the preceding claims, characterised in that one carries out the precipitation at a pH value between 2 and 9.

9. Process according to one of the preceding claims, characterised in that one dissolves the separated off precipitate with buffer solution, pH 4.5 to 10.

10. Process according to one of the preceding claims, characterised in that, from the solution obtained from the precipitate, the dyestuff is separated from the active protein by the addition of polycations or adsorbents, by precipitation of the active protein by means of organic solvents or salts or by dialysis or ultrafiltration.

**Revendications**

1. Procédé pour la purification ou l'enrichissement de protéines biologiquement actives par addition d'un agent de précipitation sélectif, caractérisé en ce qu'on additionne une solution aqueuse qui contient la protéine désirée, à une valeur de pH inférieure à son point isoélectrique, d'une quantité d'un colorant azoïque d'acide arylsulfonique avec deux groupes sulfonate qui sont séparés par 8 à 10 doubles liaisons se trouvant en relation de résonance, parmi lesquelles se trouve au moins une double liaison N = N, jusqu'à ce qu'au moins la plus grande partie de l'activité biologique de la protéine active désirée se trouve dans le précipité, en ce qu'on sépare le précipité et en ce qu'on ramène la protéine active en solution à une valeur de pH supérieure à son point isoélectrique.

2. Procédé suivant la revendication 1, caractérisé en ce qu'on utilise comme colorant azoïque l'acide 3,3′-(4,4′-biphénylylène-bis(azo)-bis(4-amino-1-naphtalènesulfonique) ou un de ses dérivés substitué par un ou plusieurs groupes alcoyle inférieur ou alcoxy inférieur.

3. Procédé suivant la revendication 2, caractérisé en ce qu'on utilise du rouge congo (C.I. 22120) ou de la benzopurpurine-10B (C.I. 23560).

4. Procédé selon la revendication 2, caractérisé en ce qu'on utilise l'acide 2,2′-[(diazoamino)-di-p-phénylène]-bis(7-benzothiazolsulfonique) ou un de ses dérivés portant un ou plusieurs groupes alcoyle inférieur et/ou alcoxy inférieur.

5. Procédé selon la revendication 4, caractérisé en ce qu'on utilise du jaune de thiazol G (C.I. 19540).

6. Procédé selon l'une des revendications précédentes, caractérisé en ce qu'on ajoute 0,01 à 2,0% en poids/volume de colorant.

7. Procédé suivant l'une des revendications précédentes, caractérisé en ce qu'on ajoute le colorant azoïque sous la forme d'une solution à 0,05 à 5%.

8. Procédé suivant l'une des revendications précédentes, caractérisé en ce qu'on effectue la précipitation à des valeurs de pH entre 2 et 9.

9. Procédé suivant l'une des revendications précédentes, caractérisé en ce qu'on dissout le précipité séparé avec une solution tampon, pH 4,5 à 10.

10. Procédé suivant l'une des revendications précédentes, caractérisé en ce qu'on sépare le colorant de la solution obtenue à partir du précipité par addition de polycations ou d'adsorbants, par précipitation de la protéine active au moyen de solvants organiques ou de sels ou par dialyse ou ultrafiltration de la protéine active.